(19) Europäisches Patentamt
European Patent Office
Office européen des brevets

(11) Publication number: 0 160 322
B1

(12) # EUROPEAN PATENT SPECIFICATION

(45) Date of publication of patent specification: 11.01.89

(51) Int. Cl.[4]: **C 07 C 79/12**, C 07 D 319/06, C 07 C 49/467, C 07 C 49/813

(21) Application number: 85105409.8

(22) Date of filing: 03.05.85

(54) **Iodonium ylide compositions and method for antimicrobial use.**

(30) Priority: 04.05.84 US 607022

(43) Date of publication of application: 06.11.85 Bulletin 85/45

(45) Publication of the grant of the patent: 11.01.89 Bulletin 89/02

(84) Designated Contracting States: AT BE CH DE FR GB IT LI NL SE

(56) References cited:
DE-A-2 166 953
US-A-3 944 498
US-A-4 450 360

CHEMICAL ABSTRACTS, vol. 69, no. 1, 1 July 1968, Columbus, Ohio (US); B.KARELE et al.: "Iodonium derivatives of beta-dicarbonyl compounds. XI. Synthesis and properties of some nitrophenyliodonium betaines and dimedone and malonic acid esters", pp. 247-248, no. 2626g

CHEMICAL ABSTRACTS, vol. 85, no. 3, 19 July 1976, Columbus, Ohio (US); B.KARELE et al.: "Iodonium derivatives of beta-dicarbonyl compounds. XIII. Synthesis and properties of some methylphenyliodonium betaines of beta-dicarbonyl compounds", p. 636, no. 20763j

(73) Proprietor: THE DOW CHEMICAL COMPANY
2030 Dow Center Abbott Road P.O. Box 1967
Midland Michigan 48640-1967 (US)

(72) Inventor: Relenyi, Attila G.
516 Bark Lane
Midland, MI 48640 (US)
Inventor: Walter, Richard W.
26 Lexington Court
Midland, MI 48640 (US)
Inventor: Koser, Gerald F.
96 Oakhurst Drive
Munroe Falls, OH 44262 (US)

(74) Representative: Weickmann, Heinrich, Dipl.-Ing. et al
Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 160 322 B1

**Description**

The present invention is directed to a method of inhibiting the growth of bacteria, algae and fungi and to antimicrobial compositions containing certain polyvalent iodonium ylide compounds as antimicrobial agents. The compounds employed in the method and composition of this invention advantageously have the formula

$$Ar-\overset{\oplus}{I}-R^1$$

as defined in the claims, wherein Ar is an optionally substituted phenyl, naphthyl, anthryl or phenanthryl group wherein the substituents on Ar may be from 0 to 3 aryl, alkyl, alkoxy, nitro or halo groups. Desirably, the compounds are represented by the formula

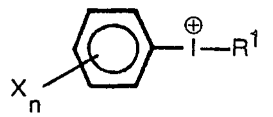 (I)

wherein X is aryl, alkyl, alkoxy, nitro or halo; n is an integer of from 0 to 3, both inclusive; and $R^1$ is

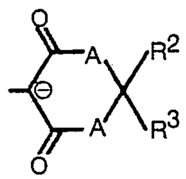 , 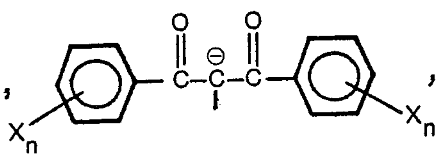 , 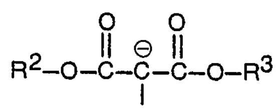 , or 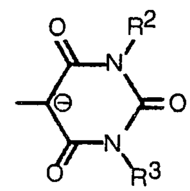

wherein A is oxygen or methylene; $R^2$ and $R^3$ may be the same or different and may be hydrogen, aryl or alkyl; and X and n have the same meaning as previously defined.

Certain of the compounds employed in the method and composition of this invention may exist as complexes, e.g. hydrates and alcoholates. Such complexes are included in the scope of this invention.

As used herein the term "aryl" refers to aromatic hydrocarbon radicals such as, for example, phenyl, naphthyl and anthryl; the term "alkyl" refers to aliphatic, straight or branched chain radicals of from one to four carbon atoms such as methyl, ethyl, propyl, isopropyl, butyl and secondary-butyl; the term "alkoxy" refers to alkoxy groups containing from one to four carbon atoms such as methoxy, ethoxy, propoxy and isopropoxy; the term "halo" refers to fluoro, chloro, bromo or iodo.

In preferred compositions and methods, the aryl moiety of the compounds employed are substituted adjacent to the bond to the iodine atom with a nitro group. More preferred for use in the method of the present invention are those compounds of formula I represented by the formula

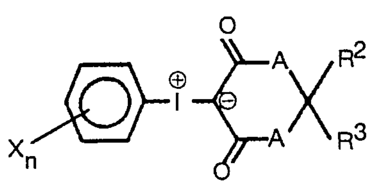

wherein X is nitro, n is the integer one or two; A is oxygen; and $R^2$ and $R^3$ are the same alkyl of one to four carbon atoms, both inclusive. Of the preferred compounds, the compound which is especially preferred for use herein is the compound wherein $R^2$ and $R^3$ are methyl, n is the integer one and X is ortho-nitro (i.e., o-nitrophenylisopropylidenemalonyliodone).

The compounds used in the method of the present invention are prepared using procedures known to the art. The methods and materials used to prepare these compounds are taught, for example, in *Structure Elucidation, Mechanism and Synthetic Applications of Organoiodone (III) Compounds: Dibenziodoles, Dibenziodolium Salts, Phenyldimedonyliodones, Phenylhydroxytosyloxyiodine and Phenylmethoxytosyloxyiodine* (A. G. Relenyi, 1982, University of Akron Library, Akron, Ohio); O. Neiland and B. Karele, *J. Org. Chem. USSR (Engl. Transl.), 7,* 1674-1677 (1971); and *The Chemistry of Functional Groups, Supplement D* (Patai and Rappoport, editors), John Wiley and Sons, Ltd., 1983.

For example, those compounds of formula I wherein $R^1$ is

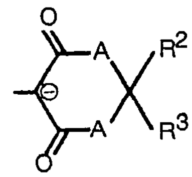

and A is oxygen may be prepared by the following procedure. Malonic acid is reacted with the necessary $R^2$, $R^3$-substituted ketone in the presence of a strong acid catalyst such as sulfuric acid or hydrochloric acid to form the desired malonate; this reaction is illustrated by the following reaction sequence:

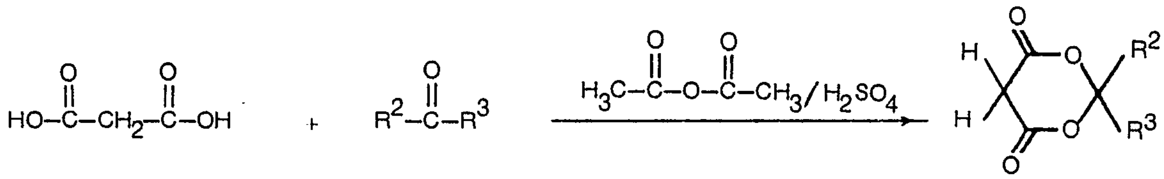

The malonate is then reacted with X-substituted iodosobenzene in an inert organic solvent such as chloroform, dichloromethane or 1,1,2,2-tetrachloroethylene; this reaction is illustrated by the following reaction sequence:

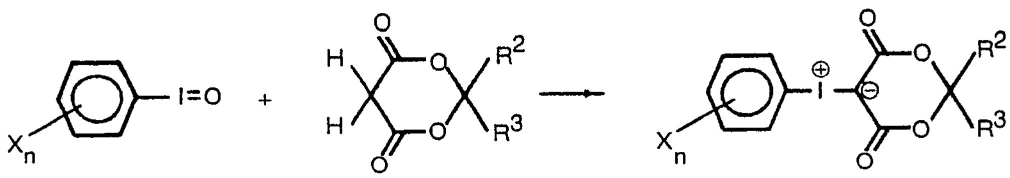

Similarly, the other compounds represented by formula I wherein $R^1$ is as defined above may be prepared in an analogous manner as, for example, by the reaction of the desired X-substituted iodosobenzene with a 1,3-dicarbonyl cyclic or acyclic compound. For instance, for those compounds of formula I wherein $R^1$ is

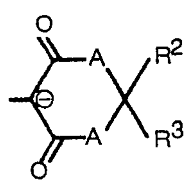

(and A is methylene and $R^2$ and $R^3$ are as defined above) the desired $R^2$, $R^3$-substituted-3,5-diketocyclohexane is reacted with X-substituted iodosobenzene in an inert organic solvent such as chloroform; this reaction is illustrated by the following reaction sequence:

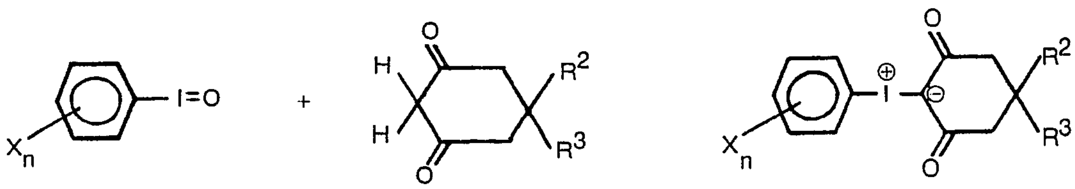

It has heretofore been unknown that the compounds of formula I, can be used in valuable antimicrobial applications (i.e., as a bactericide, fungicide or algaecide). For example, the compounds of formula I or compositions containing one or more of them as the active antimicrobial constituent can be incorporated into or upon plaster, ink, wallboard, textiles, paper, adhesives, soaps, synthetic detergents, cutting oils, polymeric materials, embalming fluids, oil-base paints, latex paints and any other aqueous based system in order to prevent the attack of various microbial pests and thus avoid the resultant economic loss due to the degradation of such products by the microorganisms. Also the compounds can be distributed in textiles, cellulosic materials or in grain or can be employed in the impregnation of wood and lumber to preserve and protect such products from the attack of the organisms of rot, mold and decay.

A preferred antimicrobial application for the compounds of formula I includes incorporation into various latexes such as adhesives, textiles, paints, papers and the like which may be subject to microbiological contamination and/or deterioration. Similarly, another preferred antimicrobial application for the compounds of formula I is as a preservative in metalworking fluids.

Still another preferred antimicrobial application for the compounds of formula I is in the prevention of

slime accumulation in water cooling towers. These compounds typically have low minimum inhibitory concentrations against aqueous-borne biofoulants often found in industrial cooling towers such as, for example, *Pseudomonas aeruginosa* and *Enterobacter aerogenes.*

Further, the compounds typically exhibit good hydrolytic stability (half-life of days to months) and are thus persistent in aqueous media. Because of the low concentrations needed to inhibit slime buildup in this environment, subsequent degradation products will also be present in low concentrations. Especially preferred for use in the water cooling tower application described above is the compound o-nitrophenylisopropylidenemalonyliodone.

The antimicrobial compositions of this invention advantageously contain from 1 to 50 percent by weight of the iodonium ylide compound and preferably contain from 1 to 20 percent by weight of said compound. In the method of this invention the bacteria, fungi or algae, or the habitat thereof, is contacted with a composition comprising from 0.00001 to 10 percent by weight (1 to 100,000 ppm), preferably 0.0001 to 5 percent by weight and most preferably from 0.0001 to 0.3 percent by weight of at least one of the iodonium ylide compounds.

The following examples further illustrate the present invention.

Example 1

o-Nitroiodosobenzene Dichloride

5.38 Grams (g) of o-nitroiodobenzene was mixed with 50 milliliters (ml) of chloroform. To this mixture was added condensed (condensed at Dry-Ice temperature) chlorine gas while the temperature was maintained at 25°C. A yellow precipitate formed which was collected by filtration giving 5.66 g of o-nitroiodosobenzene dichloride, melting point (m.p.) 82°—84°C.

Example 2

o-Nitroiodosobenzene

o-Nitroiodosobenzene dichloride (3.0 g) was triturated with 15 ml of 33% potassium hydroxide solution. An additional 20 ml of the aqueous potassium hydroxide solution was added, and the resulting orange powder was collected by filtration, washed with water and then three times with 20 ml portions of diethyl ether, giving 1.78 g of o-nitroiosobenzene.

Example 3

Isopropylidene Malonate

Following the general method of Davidson and Bernhard (J. Amer. Chem. Soc., *70,* 3426 (1948)) isopropylidene malonate was prepared as follows: Malonic acid (26.08 g) was mixed with acetic anhydride (31 ml) and then 1.58 g of concentrated sulfuric acid was added whereupon some of the malonic acid dissolved. The resultant mixture was cooled in an ice bath and 21 ml of acetone was added with stirring keeping the temperature below 20°C. The solution was cooled to about 0°C for about 4 days during which time a yellow color developed and the solution froze. During the 4 day period, the frozen mixture was occasionally thawed, stirred, and allowed to refreeze. After the 4 day period, a solid was recovered from the cold mixture. The solid was washed with 250 ml of ice cold water (about 3°—5°C) and allowed to dry in air to give 21.11 g of white crystalline isopropylidene malonate. The isopropylidene malonate should preferably be stored under refrigeration.

Example 4

o-Nitrophenylisopropylidenemalonyliodone

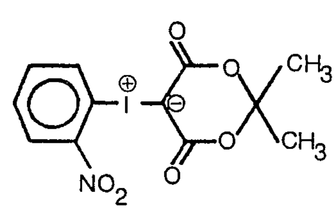

To a mixture of isopropylidene malonate (0.917 g) in 20 ml of chloroform was added (in small increments initially) 1.78 g of o-nitroiodosobenzene. This mixture was then stirred for about 15 minutes resulting in a precipitate which was collected by filtration. The precipitate was washed with diethyl ether and then dried leaving 1.88 g of a light yellow powder identified as o-nitrophenylisopropylidenemalonyliodone, m.p. 166°—168°C.

Example 5

p-Nitroiodosobenzene Dichloride

Condensed chlorine gas was added dropwise for 10 minutes to a cold (approximately −30°C) mixture of 20 g of p-nitroiodobenzene in 300 ml of chloroform resulting in the formation of fine yellow crystals which were recovered by filtration. After prolonged cooling of the above mixture, an additional crop of

crystals was obtained by filtration. Based on the melting point and known method of preparation, the product (22.09 g) was identified as p-nitroiodosobenzene dichloride, m.p. 148°C.

Example 6

p-Nitroiodosobenzene

A mixture of p-nitroiodobenzene (5.00 g) and acetonitrile (about 45 ml) was treated with condensed chlorine gas (3 ml) to give p-nitroiodosobenzene dichloride as fine yellow crystals. The p-nitroiodosobenzene dichloride (3.83 g) was thoroughly ground with 90 ml of concentrated aqueous sodium hydroxide solution added in three 30 ml portions to give a bright orange paste which was collected. The collected material was washed with water, filtered, and then washed with chloroform, filtered, and allowed to dry in air for several hours to give 3.03 g of a yellow solid (m.p. 87°C, exploded) identified as p-nitroiodosobenzene.

Example 7

p-Chloroiodosobenzene Diacetate

A cold (0°—10°C) solution consisting of p-chloroiodobenzene (24.91 g) of 150 ml of chloroform was treated with condensed chlorine gas and a bright yellow precipitate, p-chloroiodosobenzene dichloride, formed. After stirring the above mixture for 15 minutes, the p-chloroiodosobenzene dichloride was isolated by filtration. The p-chloroiodosobenzene dichloride was then dissolved in glacial acetic acid (150 ml) and this mixture cooled to near freezing and then treated with 200 ml of a solution of lead acetate (39.63 g) in glacial acetic acid. After one-half hour of vigorous stirring, the reaction mixture was filtered (to remove lead chloride), and the filtrate was poured onto about 500 g of cracked ice. The resultant aqueous mixture was extracted four times with 400 ml portions of dichloromethane. The dichloromethane extracts were combined and this dichloromethane solution was washed three times with 300 ml portions of water, dried over magnesium sulfate and evaporated to dryness to give 17.70 g of p-chloroiodosobenzene diacetate.

Example 8

p-Methoxyiodosobenzene Diacetate

Condensed chlorine gas (5 ml) was added to a solution of p-methoxyiodobenzene (about 6 g) in 100 ml of chloroform. A bright yellow solid, p-methoxyiodosobenzene dichloride, precipitated. The p-methoxyiodosobenzene dichloride product was quickly filtered from the reaction mixture and was not allowed to dry (to prevent decomposition). The chloroform-damp product was quickly added to a solution of lead acetate (16.42 g) in 130 ml of glacial acetic acid and stirred. After the reaction mixture had been stirred for two hours, the lead chloride which had formed was removed by filtration and washed with acetic acid (50 ml). The acetic acid wash was combined with the reaction mixture filtrate (i.e., the reaction mixture from which the lead chloride was removed) and this mixture was poured onto about 500 g of cracked ice which resulted in the formation of a precipitate. The precipitate was recovered by filtration and air dried to give 1.33 g of white, crude p-methoxyiodosobenzene diacetate (m.p. 74°—81°C). After removal of the p-methoxyiodosobenzene diacetate that had precipitated, the filtrate (an aqueous acetic acid-chloroform mixture) was extracted with dichloromethane (six times with 200 ml portions). The dichloromethane extracts were combined then dried over magnesium sulfate and then concentrated giving an oil. The oil was subjected to continuous Soxhlet extraction for 43 hours with a solution of acetone (30 ml) and n-hexane (150 ml). A powder was formed in the extraction thimble which was filtered and dried to give 5.93 g of p-methoxyiodosobenzene diacetate, m.p. 82°—83.5°C.

Example 9

Phenyldimedonyliodone

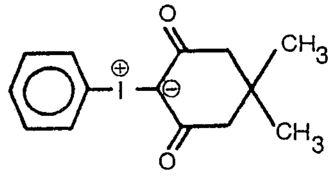

10.00 grams of iodosobenzene diacetate (prepared in a manner analogous to that described herein) and 4.35 g of dimedone (i.e., 1,1-dimethyl-3,5-diketocyclohexane) were stirred for 30 minutes in 250 ml of dichloromethane. This reaction mixture was then washed three times with 100 ml portions of 5 percent aqueous sodium hydroxide. The organic layer was then washed three times with 100 ml portions of a saturated aqueous sodium chloride solution, dried over magnesium sulfate, and the dichloromethane was then evaporated leaving a white, flaky, amorphous solid. The solid was vigorously stirred with diethyl ether, and dried to give 8.13 g of phenyldimedonyliodone, m.p. 128.5°—129.5°C.

Example 10

p-Methoxyphenyldimedonyliodone

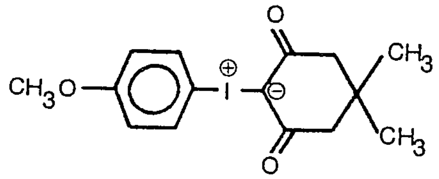

A cold (0°C) mixture of p-methoxyiodosobenzene diacetate (6.0 g) and dimedone (2.40 g) in 75 ml of chloroform was stirred for one-half hour, then warmed to room temperature and stirred for an additional 1.5 hours. The mixture was then evaporated to dryness (at approximately 30°C, aspirator vacuum) and the residue was triturated with diethyl ether (100 ml) to give 4.76 g of a white powder after filtration (m.p. 147°—151°C). The resultant white powder was dissolved in chloroform (30 ml) and then diethyl ether was added (100 ml). Upon slow evaporation, a slurry was obtained which was filtered, and 3.42 g of a precipitate recovered. The precipitate was identified as p-methoxyphenyldimedonyliodone, m.p. 145°—147°C.

Example 11

p-Nitrophenyldimedonyliodone

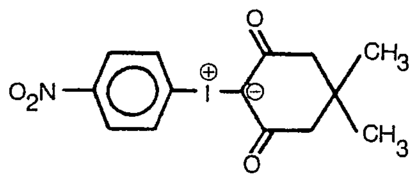

Dimedone (1.60 g) and p-nitroiodosobenzene (3.03 g) were added to 20 ml of chloroform, and the resulting mixture was stirred overnight. A precipitate formed which was isolated by filtration giving 2.22 g of a white flaky solid after air drying (m.p. 130°—131°C). Diethyl ether (60 ml) was added to the filtrate and a white, flaky precipitate formed which was isolated and dried (1.17 g; m.p. 130°—131°C). The white solids were combined and subjected to continuous extraction with diethyl ether to give 2.96 g of p-nitrophenyldimedonyliodone (m.p. 130°—131°C).

Example 12

p-Chlorophenyldimedonyliodone

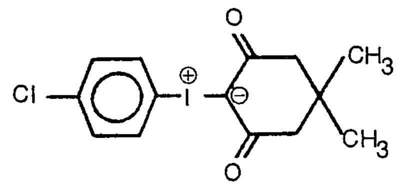

Dimedone (6.95 g) was added to a slurry of p-chloroiodosobenzene diacetate (17.70 g) in 100 ml of dichloromethane. After stirring for one-half hour, the mixture gelled. The gelled mixture was filtered to give an amorphous solid. The solid was washed three times with 100 ml portions of dichloromethane to give 15.72 g of a white, flaky solid, identified as p-chlorophenyldimedonyliodone, m.p. 157°—158°C.

Example 13

p-Bromophenyldimedonyliodone

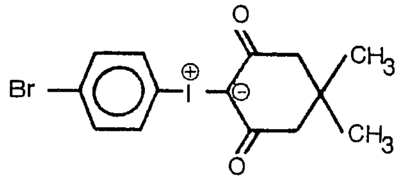

A solution of 50.95 g of p-bromoiodobenzene in 150 ml of chloroform was treated with condensed

chlorine gas which gave a fine yellow precipitate of p-bromoiodosobenzene dichloride. The p-bromoiodosobenzene dichloride precipitate was removed by filtration and washed with cold chloroform. To a cold (0°C) solution of the p-bromoiodosobenzene dichloride in 900 ml of acetonitrile was added 166.92 g of silver acetate. After a few minutes, the silver chloride which precipitated was removed by filtration and the filtrate was concentrated to a slurry. The slurry was then dissolved in 1000 ml of chloroform, filtered, and 25.33 g of dimedone added. After about 15 minutes a white precipitate formed in the mixture and the mixture was then cooled at 0°C for 24 hours. After the cooling period, the precipitate was recovered by filtration and 29.66 g of a white flaky solid was recovered. The white flaky solid was washed three times with 200 ml portions of chloroform and then dried. Diethyl ether (200 ml) was added to the filtrate which precipitated an additional quantity (18.36 g) of white, flaky material. The combined precipitates were identified as p-bromophenyldimedonyliodone, m.p. 161°—162°C.

*Analysis:*

| | | | |
|---|---|---|---|
| Calculated for $C_{14}H_{14}BrIO_2$: | C, 39.94; | H, 3.35; | I, 30.14 |
| Found: | C, 39.78; | H, 3.43; | I, 29.97 |

Example 14

Phenyldibenzoylmethyliodone

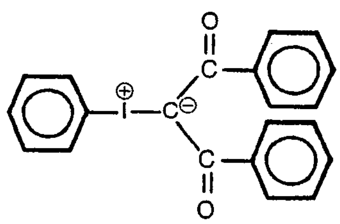

Potassium hydroxide (17.0 g) in 500 ml of methanol was cooled in an ice bath to 0°—5°C and dibenzoylmethane (22.4 g) was added. Iodosobenzene diacetate (32.2 g) was slowly added to the cold mixture. After stirring for one hour, 500 ml of an ice-water mixture was slowly added to promote crystal formation. The resultant crystal crop was washed with two 100 ml portions of methanol and then rinsed with diethyl ether. The resultant solid was dissolved in chloroform and reprecipitated with petroleum ether to give 20.0 g (23.5% yield) of dark yellow crystalline phenyldibenzoylmethyliodone, m.p. 85°—88°C.

Example 15

p-Biphenylyldimedonyliodone

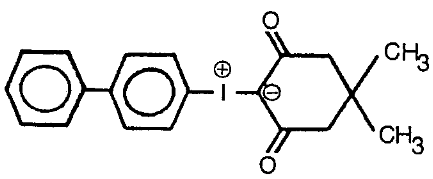

p-Iodobiphenyl (10.0 g) was dissolved in 100 ml chloroform. As the chloroform solution was cooled to Dry-Ice/acetone temperature, chlorine gas was bubbled into the solution. After a few minutes of treatment with a steady stream of chlorine gas, the resultant yellow solution was allowed to warm to room temperature. To promote crystal growth, the sides of the flask were scratched with a sharp unpolished glass rod and a fine yellow precipitate formed which was isolated by filtration. The crystals were dried in air to give 10.3 g of p-biphenylyliodosodichloride. The p-biphenylyliodosodichloride was added to a mixture consisting of 2.08 g dimedone and 125 ml chloroform. After about 10 minutes of stirring, 20 ml of aqueous dilute sodium hydroxide solution was added. A clear chloroform layer and a clear aqueous layer formed after stirring for a few minutes. The chloroform layer was separated, which upon subsequent treatment with hexanes gave an off-white precipitate which was recovered by filtration and dried in air to give 2.31 g of p-biphenylyldimedonyliodone, m.p. 147°—148°C.

The antimicrobial activity of the compounds of formula I was demonstrated by the following techniques.

The minimum inhibitory concentration (MIC) for the compound of Example 4 (i.e., o-nitrophenylisopropylidenemalonyliodone) was determined for 9 bacteria (using nutrient agar) and 5 yeast and fungi (using malt yeast agar). A one percent solution of this test compound (i.e., the compound of Example 4) was prepared in a mixture of acetone-water. Nutrient agar was prepared at pH 6.8 using deionized water according to standard Difco procedures. Malt yeast agar was prepared by adding 3 g Bacto yeast extract and 45 g Bacto Malt agar per liter of deionized water. The agar (nutrient agar when testing with bacteria and malt yeast agar when testing with yeast and fungi) was dispensed in 30 ml aliquots into

25 × 200 millimeter (mm) test tubes, capped and autoclaved for 15 minutes at 115°C. The test tubes containing the agar were cooled in a water bath until the temperature of the agar was 48°C and then an appropriate amount of the one percent solution of the test compound was added (except in the controls where no test compound was added) to the respective test tubes so that final concentrations of 500, 250, 100, 50, 25, 10, 5, 2.5, 1.0 and 0 parts per million (ppm) of the test compound in the agar were obtained. The agar solutions were each mixed and poured into individual petrie plates so that each petrie plate contained agar having a known concentration of test compound dispersed therein. After drying for 24 hours, the petrie plates were inoculated with bacteria when the petrie plates contained nutrient broth agar or with fungi and yeast when the petrie plates containing malt yeast agar.

The inoculation with bacteria was accomplished using the following procedure. Twenty-four hour cultures of each of the bacteria were prepared by incubating the respective bacteria in tubes containing nutrient broth for 24 hours at 30°C in a shaker. Dilutions of each of the 24 hour cultures were made so that 9 separate suspensions (one for each of the 9 test bacteria) were made, each containing $10^8$ colony forming units (CFU) per ml of suspension of a particular bacteria. Aliquots of 0.3 ml of each of the above suspensions were used to fill individual wells of a Steer's Replicator. For each microbial suspension, 0.3 ml was used to fill 3 wells (i.e., 3 wells of 0.3 ml each) so that for the 9 different bacteria 27 wells were filled. The Steer's Replicator was then used to inoculate the petrie plates.

The petrie plates were incubated at 30°C for 48 hours and then read to determine if the test compound (i.e., the compound of Example 4) which was incorporated into the agar prevented growth of the respective bacteria. The minimum inhibitory concentration (MIC) for each bacteria was defined as the lowest concentration of the test compound which prevented growth of that bacteria.

The inoculation with the fungi and yeast was accomplished as follows. Cultures of fungi and yeast were incubated for 7 days on malt yeast agar at 30°C. These cultures were used to prepare suspensions by the following procedure. A suspension of each organism was prepared by adding 10 ml of sterile saline and 10 microliters (µl) of Triton X100 to the slant. The sterile saline/Triton X100 solution was then agitated with a sterile swab to suspend the microorganism grown on the slant. Each resulting suspension was diluted into sterile saline (1 part suspension: 9 parts sterile saline). Aliquots of these dilutions were placed in individual wells of a Steer's Replicator and petrie plates inoculated as previously described. The petrie plates were incubated at 30°C and read after 48 hours for yeast and 72 hours for fungi.

Table 1 sets forth the MIC (in ppm) of o-nitrophenylisopropylidenemalonyliodone for the organisms shown therein.

TABLE 1

| Organism | ATCC # | MIC (o-nitrophenylisopropylidenemalonyliodone) |
|---|---|---|
| Bacillus subtilis | 8473 | ≤1.0 |
| Enterobacter aerogenes | 13048 | 1.0 |
| Escherichia coli | 11229 | ≤1.0 |
| Klebsiella pneumoniae | 8308 | ≤1.0 |
| Proteus vulgaris | 881 | ≤1.0 |
| Pseudomonas aeruginosa | 10145 | 10 |
| Pseudomonas aeruginosa PRD-10 | 15442 | 10 |
| Salmonella choleraesuis | 10708 | ≤1.0 |
| Staphylococcus aureus | 6538 | ≤1.0 |
| Aspergillus niger | 16404 | 250 |
| Penicillium chrysogenum | 9480 | 100 |
| Trichoderma viride | 8678 | 250 |
| Candida albicans | 10231 | 100 |
| Saccharomyces cervisiae | 4105 | 100 |

In a similar procedure, the MIC (in ppm) of various compounds of formula I were determined for the organism *Enterobacter aerogenes* in a neutral solution (pH = 6.8) and in an alkaline medium (pH = 8.2). These results are set forth in Table 2.

TABLE 2

| Compound Example No. | MIC NEA[a] | MIC AEA[b] |
|---|---|---|
| 4 | ≤1.0 | 2.5 |
| 9. | 2500 | 2000 |
| 10 | 1500 | 1500 |
| 11 | 1000 | 750 |
| 12 | 2000 | 1500 |
| 13 | 1000 | 1000 |
| 14 | 1500 | 1500 |
| 15 | 1500 | 1500 |

[a]*Enterobacter aerogenes* in neutral growth medium (pH = 6.8)

[b]*Enterobacter aerogenes* in an alkaline growth medium (pH = 8.2)

The ability of the compounds of formula I to serve as preservatives (as demonstrated by the use of o-nitrophenylisopropylidenemalonyliodone) was tested both in latex and cutting oil emulsions. The latex was a vinylidene chloride-butadiene copolymer with a pH of 6.1. The emulsion type cutting oil was a concentrate, Vantrol® 51—086—B, manufactured by Van Straaten Chemical Company; this concentrate was diluted 1:40 with tap water and had a final pH of 9.7.

Fifty gram aliquots of the latex were placed in sterile bottles and one hundred gram aliquots of the diluted cutting oil were placed in 250-ml Erlenmeyer flasks. An appropriate amount of a fresh 1% stock solution of o-nitrophenylisopropylidenemalonyliodone in acetone-water was added to achieve the desired final concentrations (see Tables 4 and 5). A small portion of the respective latex or cutting oil preparation was then streaked onto Tryptic Soy Agar (TSA) petri plates using sterile cotton swabs to determine whether the preparations were sterile. If the particular preparation was sterile, it was then inoculated with an appropriate volume (0.1 ml for the latex and 0.2 ml for the cutting oil) of a mixture of equal aliquots of 24 hour cultures of each of the bacterial organisms listed in Table 1 (not the fungi and yeast). The latex samples were incubated at 30°C and the cutting oils were agitated on a rotary shaker at room temperature. After 24 hours, all samples were again streaked on TSA. All plates were then incubated at 30°C for 48 hours and then rated 1 to 10 according to the growth rating system described in Table 3.

TABLE 3

Growth Rating

| Rating | No. of Colonies |
|---|---|
| 1 | 0 |
| 2 | 1-4 |
| 3 | 5-10 |
| 4 | 11-25 |
| 5 | 26-50 |
| 6 | 51-100 |
| 7 | 101-200 |
| 8 | 201-300 |
| 9 | Too many to count |
| 10 | Solid mass |

The results from this first set of streaks after inoculation are listed in the columns labeled streak number 1 in Tables 4 and 5. Samples with a rating of 3 or less from streak number 1 were reinoculated as described for the first inoculation above. Samples with a rating of 4 or greater were not reinoculated. After another 24 hours all samples were again restreaked on TSA agar. The results from these second streaks were labeled streak number 2 in Tables 4 and 5. Samples were reinoculated and restreaked in this fashion for a maximum of 10 streaks. Any samples with two streaks in a row with a ten rating were not restreaked again.

TABLE 4

Latex Preservation Test[a]

| Conc. | Streak Number | | | | | | | | | |
|---|---|---|---|---|---|---|---|---|---|---|
| (ppm) | 1 | 2 | 3 | 4 | 5 | 6 | 7 | 8 | 9 | 10 |
| 1000 | 1 | 1 | 1 | 1 | 1 | 1 | 1 | 1 | 1 | 1 |
| 500 | 1 | 1 | 1 | 1 | 1 | 1 | 1 | 1 | 1 | 1 |
| 250 | 1 | 1 | 1 | 1 | 1 | 1 | 1 | 1 | 1 | 1 |
| 100 | 1 | 1 | 1 | 1 | 1 | 1 | 1 | 1 | 1 | 1 |
| 50 | 1 | 1 | 1 | 1 | 1 | 1 | 1 | 1 | 1 | 1 |
| 25 | 1 | 1 | 1 | 1 | 1 | 1 | 1 | 1 | 2 | 2 |
| 10 | 1 | 1 | 1 | 1 | 6 | 10 | 10 | 10[b] | | |
| 5 | 6 | 1 | 7 | 10 | 10 | 10[b] | | | | |

[a]The values shown represent the growth rating obtained (see Table 3) for each particular challenge when o-nitrophenylisopropylidenemalonyliodone was present at the indicated concentration.

[b]Restreaking was not continued.

TABLE 5

Cutting Oil Preservation Test[a]

| Conc. | Streak Number | | | | | | | | | |
|---|---|---|---|---|---|---|---|---|---|---|
| (ppm) | 1 | 2 | 3 | 4 | 5 | 6 | 7 | 8 | 9 | 10 |
| 1000 | 1 | 1 | 1 | 1 | 1 | 1 | 1 | 1 | 1 | 1 |
| 500 | 1 | 1 | 1 | 1 | 1 | 1 | 1 | 1 | 1 | 1 |
| 250 | 1 | 1 | 1 | 1 | 1 | 1 | 1 | 1 | 6 | 10 |
| 100 | 1 | 2 | 2 | 1 | 10 | 10[b] | | | | |
| 50 | 1 | 2 | 10 | 10[b] | | | | | | |
| 25 | 1 | 9 | 10 | 10[b] | | | | | | |

[a]The values shown represent the growth rating obtained (see Table 3) for each particular challenge when o-nitrophenylisopropylidenemalonyliodone was present at the indicated concentration.

[b]Restreaking was not continued.

The ability of the compounds of formula I to serve as algaecides (as demonstrated by the use of o-nitrophenylisopropylidenemalonyliodone) was evaluated in a test which measures the inhibition of growth of *Selenastrum capricornutum.* Using a Coulter Counter, the growth of this algae was measured after 96 hours incubation with various levels of biocide (i.e., o-nitrophenylisopropylidenemalonyliodone). The results of the testing are shown in Table 6.

TABLE 6

Algaecide Test[a]

| Concentration (ppm) | Percent of Control |
|---|---|
| 32 | 0.53 |
| 10 | 0.59 |
| 1.0 | 0.65 |
| 0.1 | 2.4 |
| 0.01 | 77.0 |

[a]The numbers in the "Percent of Control" column represent the percent obtained by comparing the growth of the algae in the presence of the indicated concentration of o-nitrophenylisopropylidenemalonyliodone to the growth of the algae in the control (i.e., the growth of the algae in the absence of o-nitrophenylisopropylidenemalonyliodone).

The compounds of formula I are useful because of their antimicrobial activity and can be used as antibacterial agents, antifungal agents, algaecidal agents or any combination thereof. Their effectiveness varies with the concentration of compound employed and the organism to be controlled. While not all compounds are effective at similar concentrations against the same organisms, all compounds of the present invention are useful in the antimicrobial method disclosed herein.

Examples of the bacteria and fungi controlled by effective amounts of one or more of the compounds of formula I are organisms such as *Bacillus subtilis, Pseudomonas aeruginosa, Enterobacter aerogenes, Escherichia coli, Proteus vulgaris, Staphylococcus aureus, Aspergillus niger* and *Candida albicans.* As used herein, the term "effective amount" refers to that amount of one or more of the compounds of formula I needed to exhibit either static or cidal effects on selected organisms. Typically, this amount varies from 1 to 2000 parts per million (ppm) by weight depending upon the particular compound tested and organism treated.

In the protection and preservation of inks, adhesives, soaps, plaster, wallboard, cutting oils, textiles, polymeric materials and paper, good results are obtained when the compounds are incorporated in such products in the amount of at least 0.001 percent by weight. In the preservation of wood, excellent results are obtained when the compounds are incorporated, by conventional treatment of the wood, in the amount of at least 0.01 pound per cubic foot (0.16 kg/cubic meter) of wood.

In the preservation and protection of oil and latex paints and primers against destruction caused by the growth of bacteria or fungi, the compounds of the present invention are preferably employed at concentrations of at least 0.001 percent by weight.

In such operations, an effective amount of the unmodified compounds are distributed or incorporated in adhesives, soaps, inks, wallboard, cutting oils, textiles, paper, polymeric materials, paint, lumber, wood products or growth media. However, the present method also embraces the employment of liquid or dust compositions containing the compounds. In such usage, the compounds are modified with one or a plurality of additaments or adjuvants including water, organic solvents, petroleum oils, petroleum distillates, or other liquid carriers, polymeric thickening agents, urea, surface active dispersing agents and finely divided inert solids. Depending upon the concentration of the compounds used in the compositions, such augmented compositions are adapted to be distributed in inks, adhesives, soaps, cutting oils, polymeric materials, paints, textiles, wallboard, paper, lumber or soil or upon the above-ground surfaces of plants, or to be employed as concentrates and subsequently diluted with additional liquid or solid carriers to produce the ultimate treating compositions. In compositions wherein the adjuvant is a finely divided solid, a surface active agent or the combination of a surface active agent and a liquid diluent, the carrier cooperates with the active component so as to facilitate the invention and to obtain an improved result.

The exact concentration of one or more of the compounds of formula I to be employed in the treating compositions is not critical and may vary considerably provided the required dosage of the effective agent is supplied in the ink, adhesive, soap, cutting oil, polymeric material, paint, textile, paper, wood or growth medium. The concentration of said compounds in liquid compositions generally is from 0.0001 to 3 percent by weight. Concentrations up to 10 percent by weight may be employed. In dusts, the concentrations of the compounds can be from 0.0001 to 95 percent by weight. In compositions to be employed as concentrates, the compounds of formula I can be present in a concentration of from 0.01 to 98 percent by weight. The quantity of treating composition to be applied to textiles, lumber, growth media and the like may vary considerably provided that the required dosage of active ingredients is applied in sufficient amounts of the

finished composition to adequately facilitate the penetration and distribution of said ingredients in and on, for example, textiles, lumber and growth media.

In the treatment of lumber, from 1 to 100 gallons of solvent composition containing one or more of the compounds of formula I is usually applied per 1,000 square feet (1 to 100 liters/24.543 square meters) of surface to be treated. In the pressure or vacuum treatment of lumber, sufficient composition is employed adequately to impregnate the wood.

In the preparation of dust compositions, one or more of the compounds of formula I can be admixed with any of the finely divided solids, such as pyrophyllite, talc, chalk and gypsum. In such operations, the finely divided carrier is ground or mixed with the said compounds or wet with a solution of the compounds in a volatile organic solvent. Similarly, dust compositions containing the products can be prepared using various solid surface active dispersing agents such as fuller's earth, bentonite, attapulgite and other clays. Depending upon the proportions of ingredients, these dust compositions can be employed for the control of pests or employed as concentrates and subsequently diluted with an additional solid surface active dispersing agent or with pyrophyllite, chalk, talc, gypsum and the like to obtain the desired amount of active ingredient in a composition adapted to be employed as described herein. Also, such dust compositions when employed as concentrates can be dispersed in water, with or without the aid of dispersing agents to form spray mixtures.

Further, spray compositions can be prepared by incorporating one or more of the compounds of formula I, or their liquid or dust concentrate compositions, in mixtures with surface-active dispersing agents such as an ionic or non-ionic emulsifying agent. Such spray compositions are readily employed for the control of microbes or are dispersed in liquid carriers to form diluted sprays containing the compounds in any desired amount suitable for microbial control. The choice of dispersing agents and amounts thereof employed are determined by the ability of the agents to facilitate the dispersion of the concentrate in the liquid carrier to produce the desired spray compositions.

Similarly, the compounds of formula I can be admixed with a suitable water-immiscible organic liquid and a surface-active dispersing agent to produce an emulsifiable concentrate which can be further diluted with water and oil to form spray mixtures in the form of oil-in-water emulsions. In such compositions, the carrier comprises an aqueous emulsion, i.e., a mixture of water-immiscible solvent, emulsifying agent and water. Preferred dispersing agents which can be employed in these compositions are oil-soluble and include the non-ionic emulsifiers such as the condensation products of alkylene oxides with the inorganic acids, polyoxyethylene derivatives or sorbitan esters, complex ether alcohols and the like. Suitable organic liquids which can be employed in the composition include petroleum oils and distillates, toluene, liquid halohydrocarbons and synthetic organic oils. The surface-active dispersing agents are usually employed in liquid compositions in the amount of from 0.1 to 20 percent by weight of the combined weight of the dispersing agent and active compound.

In addition, other liquid compositions containing the desired amount of one or more of the compounds of formula I can be prepared by dissolving said compounds in an organic liquid such as acetone, methylene chloride, chlorobenzene and petroleum distillates. The preferred organic solvent carriers are those which are adapted to accomplish the penetration and impregnation of the environment to be treated.

In further embodiments, the compounds as employed in accordance with the present invention, or compositions containing the same, can be advantageously employed in the methods described herein in combination with one or more pesticidal or preservative compounds. In such embodiment, such pesticidal or preservative compound is employed either as a supplemental active constituent, an additament or as an adjuvant. Representative pesticidal or preservative compounds include the substituted phenols, cresols, substituted cresols and their metal salts, the bisphenols and thiobisphenols, the halogenated salicylanilides, the organosulfur compounds, the carbamate compounds, the quaternary ammonium compounds, the organometallic compounds, the inorganic salts and miscellaneous other compounds.

## Claims

1. A method of inhibiting the growth of bacteria, fungi or algae which comprises contacting said bacteria, fungi or algae, or the habitat thereof, with an effective amount of a compound of the formula

$$\overset{\oplus}{\text{Ar-I}}\text{—R}^1$$

wherein Ar is an optionally substituted phenyl, naphthyl, anthryl or phenanthryl group wherein the substituents may be from 0 to 3 aromatic hydrocarbon radicals (aryl), $C_{1-4}$ alkyl, $C_{1-4}$ alkoxy, nitro or halo groups; and $R^1$ is

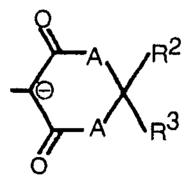
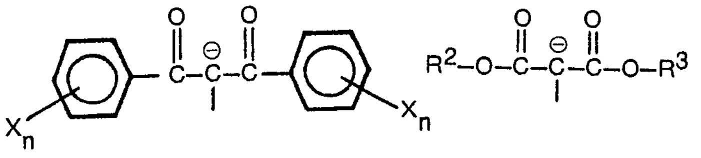
, or
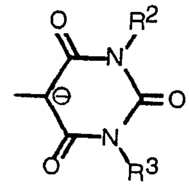

wherein A is oxygen or methylene; $R^2$ and $R^3$ may be the same or different and are hydrogen, an aromatic hydrocarbon radical (aryl) or $C_{1-4}$ alkyl; X is an aromatic hydrocarbon radical (aryl), $C_{1-4}$ alkyl, $C_{1-4}$ alkoxy, nitro or halo and n is an integer of from 0 to 3, both inclusive.

2. Method of Claim 1 wherein the compound has the formula

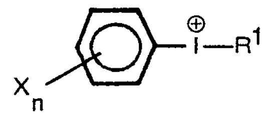

wherein $R^1$, X and n have the same meaning as defined in Claim 1.

3. Method of Claim 2 wherein the compound

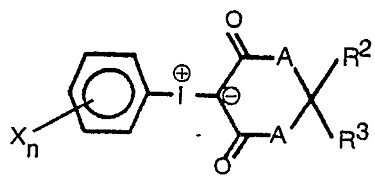

wherein X is nitro; n is the integer one or two; A is oxygen; and $R^2$ and $R^3$ are the same alkyl of one to four carbon atoms, both inclusive.

4. Method of Claim 2 wherein the compound is phenyldimedonyliodone, p-methoxyphenyldimedonyliodone, p-nitrophenyldimedonyliondone, p-chlorophenyldimedonyliodine, p-bromophenyldimedonyliodone, phenyldibenzoylmethyliodone, or p-biphenylyldimedonyliodone.

5. Method of Claim 3 wherein the compound is o-nitrophenylisopropylidenemalonyliodone having the formula

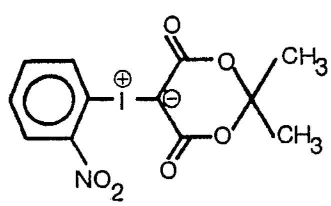
.

6. An antimicrobial composition comprising inert or antimicrobial composition adjuvants in combination with an effective amount of a compound of the formula

$$Ar\text{-}\overset{\oplus}{I}—R^1$$

wherein Ar is an optionally substituted phenyl, naphthyl, anthryl or phenanthryl group wherein the substituents may be from 0 to 3 aromatic hydrocarbon radicals (aryl), $C_{1-4}$ alkyl, $C_{1-4}$ alkoxy, nitro or halo groups; and $R^1$ is

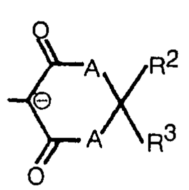
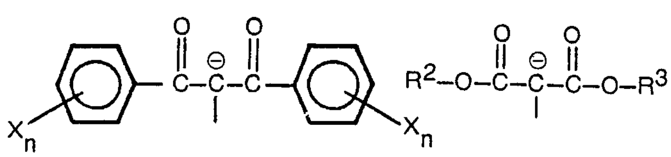
, or
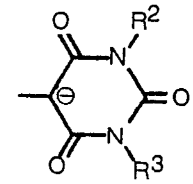

wherein A is oxygen or methylene; $R^2$ and $R^3$ may be the same or different and are hydrogen, an aromatic hydrocarbon radical (aryl) or $C_{1-4}$ alkyl; and X and n have the same meaning as defined in Claim 1.

7. Composition of Claim 6 wherein the compound has the formula

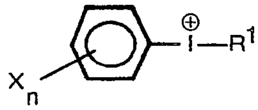

wherein X and n have the same meaning as defined in Claim 1.

8. Composition of Claim 6 wherein the compound is

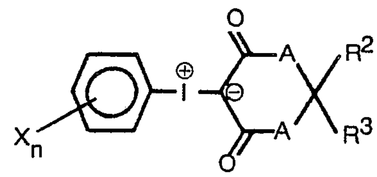

wherein X is nitro; n is the integer one or two; A is oxygen; and $R^2$ and $R^3$ are the same alkyl of one to four carbon atoms, both inclusive.

9. Composition of Claim 6 wherein the compound is phenyldimedonyliodone, p-methoxyphenyldimedonyliodone, p-nitrophenyldimedonyliodone, p-chlorophenyldimedonyliodone, p-bromophenyldimedonyliodone, phenyldibenzoylmethyliodone, or p-biphenylyldimedonyliodone.

10. Composition of Claim 8 wherein the compound is o-nitrophenylisopropylidenemalonyliodone, having the formula

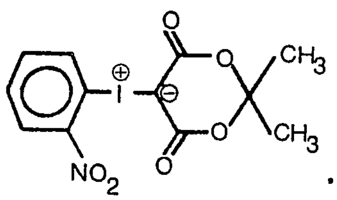

.

**Patentansprüche**

1. Verfahren zur Inhibition des Wachstums von Bakterien, Pilzen oder Algen, das Kontaktieren der Bakterien, Pilze oder Algen oder deren Lebensraumes mit einer wirksamen Menge einer Verbindung der Formel

$$Ar\text{-}\overset{\oplus}{I}\text{—}R^1$$

umfaßt, worin Ar eine gewüschtenfalls substituierte Phenyl-, Naphthyl-, Anthryl- oder Phenanthrylgruppe ist, worin die Substituenten 0 bis 3 aromatische Kohlenwasserstoffreste (Aryl), $C_{1-4}$-Alkyl-$C_{1-4}$-Alkoxy-, Nitro- oder Halogengruppen sein Können; und $R^1$

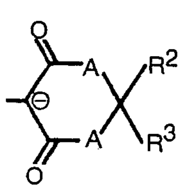 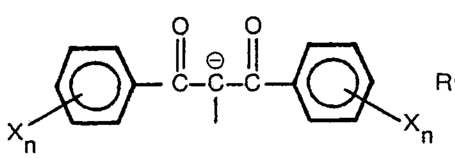 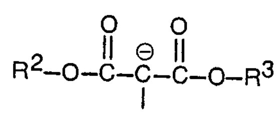 oder 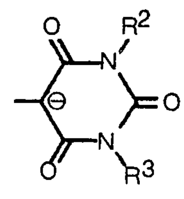

ist, worin A Sauerstoff oder Methylen ist; $R^2$ und $R^3$ gleich oder verschieden und Wasserstoff, ein aromatischer Kohlenwasserstoffrest (Aryl) oder $C_{1-4}$-Alkyl sein kann; X ein aromatischer Kohlenwasserstoffrest (Aryl), $C_{1-4}$-Alkyl, $C_{1-4}$-Alkoxy, Nitro oder Halo ist und n eine ganze Zahl vo 0 bis 3, jeweils inklusive, ist.

2. Verfahren nach Anspruch 1, worin die Verbindung die Formel

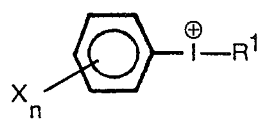

hat, worin $R^1$, X und n die gleiche Bedeutung wie im Anspruch 1 definiert, haben.

3. Verfahren nach Anspruch 2, worin die Verbindung

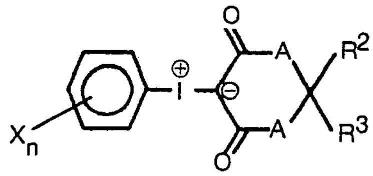

ist, worin X Nitro ist; n die ganze Zahl 1 oder 2 ist; A Sauerstoff ist; and $R^2$ and $R^3$ gas gleiche Alkyl mit 1 bis 4 Kohlenstoffatomen, jeweils inklusive, ist.

4. Verfahren nach Anspruch 2, wori die Verbindung Phenyldimedonyljodon, p-Methoxyphenyldimedonyljodon, p-Nitrophenyldimedonyljodon, p-Chlorophenyldimedonyljodon, p-Bromphenyldimedonyljodon, Phenyldibenzoylmethyljodon, oder p-Bephenylyldimedonyljodon ist.

5. Verfahren nach Anspruch 3, worin die Verbindung o-Nitrophenylisopropyliden-malonyljodon mit der Formel

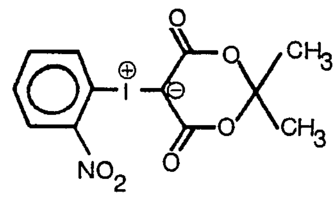

ist.

6. Antimikrobielle Zusammensetzung, umfassend inerte oder antimikrobielle Zusammensetzungshilfsstoffe in Kombination mit einer wirksamen Menge einer Verbindung der Formel

$$Ar\text{-}\overset{\oplus}{I}\text{—}R^1$$

worin Ar eine gewünschtenfalls substituierte Phenyl-, Naphthyl-, Anthryl-, oder Phenanthrylgruppe ist, worin die Substituenten von 0 bis 3 aromatische Kohlenwasserstoffreste (Aryl), $C_{1-4}$-Alkyl, $C_{1-4}$-Alkoxy-, Nitro- oder Halogrupeen sind; und $R^1$

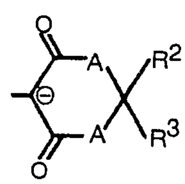 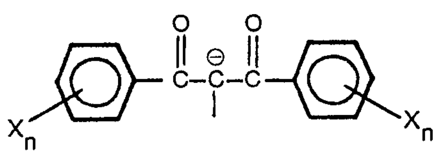 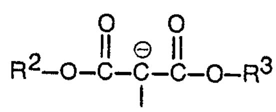 oder 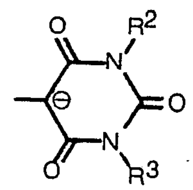

ist, worin A Sauerstoff oder Methylen ist; $R^2$ und $R^3$ gleich oder verschieden und Wasserstoff, ein aromatischer Kohlenwasserstoffrest (Aryl) oder $C_{1-4}$-Alkyl ist; und X und n die im Anspruch 1 definierte Bedeutung haben.

7. Zusammensetzung nach Anspruch 6, worin die Verbindung die Verbindung die Formel

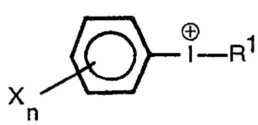

hat, worin X und n dieselbe Bedeutung wie im Anspruch 1 definiert, haben.

8. Zusammensetzung nach Anspruch 6, worin die Verbindung

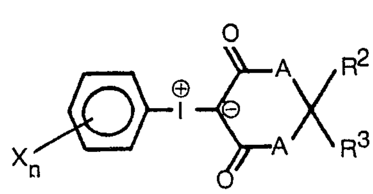

ist, worin X Nitro ist; n die ganze Zahl 1 oder 2 ist; A Sauerstoff ist und $R^2$ and $R^3$ das gleiche Alkyl mit 1 bis 4 Kohlenstoffatomen, jeweils inklusive, sind.

9. Zusammensetzung nach Anspruch 6, worin die Verbindung Phenylidimedonlyjodon, p-Methoxyphenyldimedonyljodon, p-Nitrophenyldimedonyljodon, p-Chlorphenyldimedonyljodon, p-Bromphenyldimedonyljodon, Phenyldibenzoylmethyljodon, oder p-Biphenylyldimedonyljodon ist.

10. Zusammensetzung nach Anspruch 8, worin die Verbindung o-Nitrophenylisopropylidenmalonyljodon mit der Formel

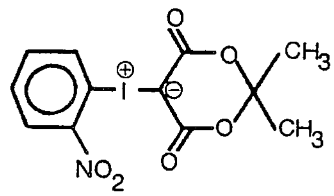

ist.

**Revendications**

1. Un procédé pour inhiber la croissance de bactéries, champignons ou algues, qui comprend la mise en contact desdits bactéries, champignons ou algues ou de leur biotope, avec une quantité efficace d'un composé de formule:

$$Ar\text{-}\overset{\oplus}{I}—R^1$$

dans laquelle Ar représente un groupement phényle, naphtyle, anthryle ou phénanthryle, éventuellement substitué, les substituants pouvant être 0 à 3 radicaux d'hydrocarbures aromatiques (argyle), groupements alkyles en $C_{1-4}$, alcoxy en $C_{1-4}$, nitro ou halogèno, et $R^1$ représente

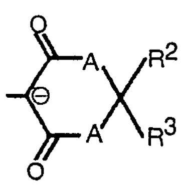 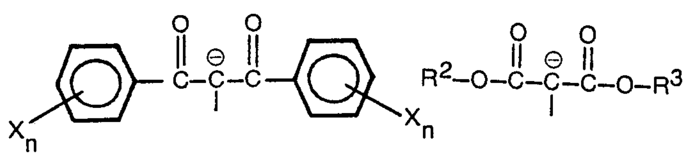 , ou 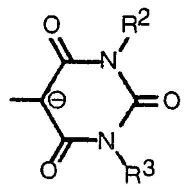

dans lesquels A représente un oxygène ou un méthylène; $R^2$ et $R^3$ peuvent être identiques ou différents et représentent un hydrogène, un radical d'hydrocarbure aromatique (aryle) ou alkyle en $C_{1-4}$; X représente un radical d'hydrocarbure aromatique (aryle), alkyle en $C_{1-4}$, alcoxy en $C_{1-4}$, nitro ou halogèno, et n est un entier compris entre 0 et 3, bornes incluses.

2. Procédé de la revendication 1, dans lequel le composé présente la formule

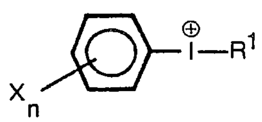

dans laquelle $R^1$, X et n ont la même signification que dans la revendication 1.

3. Procédé de revendication 2, dans lequel le composé est

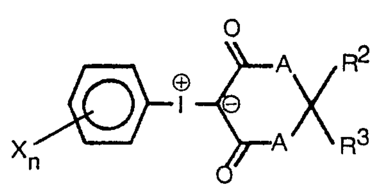

dans lequel X signifie nitro; n est l'entier 1 ou 2; A est un oxygène; et $R^2$ et $R^3$ représentent le même alkyle comprenant 1 à 4 atomes de carbone, bornes incluses.

4. Procédé de la revendication 2, dans lequel le composé est la phényldimédonyliodone, p-méthoxyphényldimédonyliodone, p-nitrophényldimédonyliodone, p-chlorophényldimédonyliodone, p-bromophényldimédonyliodone, phényldibenzoylméthyliodone ou p-biphénylyldimédonyliodone.

5. Procédé de la revendication 3, dans lequel le composé est l'o-nitrophénylisopropylidènemalonyliodone de formule

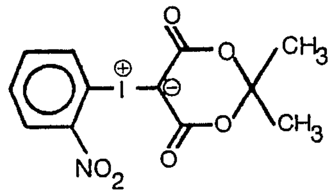

6. Une composition antimicrobienne renfermant des adjuvants inertes ou antimicrobiens de composition, combinés à une quantité efficace d'un composé de formule

$$Ar\text{-}\overset{\oplus}{I}\text{—}R^1$$

dans laquelle Ar représente un groupement phényle, naphtyle, anthryle ou phénanthryle, éventuellment substitué, les substituants pouvant être 0 à 3 radicaux d'hydrocarbures aromatiques (aryle), groupements alkyles en $C_{1-4}$, alcoxy en $C_{1-4}$, nitro ou halogèno, et $R^1$ représente

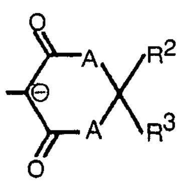 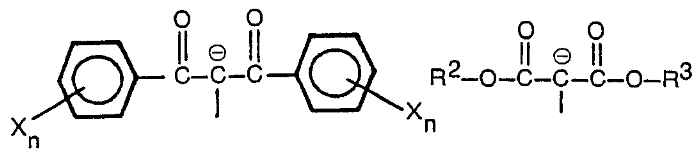 , ou 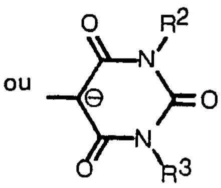

dans lesquel A représente un oxygène ou un méthylène; $R^2$ et $R^3$ peuvent être identiques ou différents et représentent un hydrogène, un radical d'hydrocarbure aromatique (argyle) ou alkyle en $C_{1-4}$; et X et n ont la même signification que dans la revendication 1.

7. Composition de la revendication 6, dans laquelle le composé présente la formule

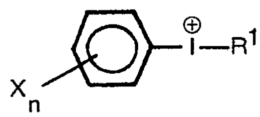

dans laquelle X et n ont la même signification que dans la revendication 1.

8. Composition de la revendication 6, dans laquelle la composé représente

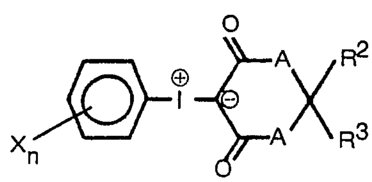

dans lequel X signifie nitro; n est l'entier 1 ou 2; A signifie un oxygène; et $R^2$ et $R^3$ représentent le même alkyle comprenant 1 à 4 atomes de carbone, bornes incluses.

9. Composition de la revendication 6, dans laquelle le composé est la phényldimédonyliodone, p-méthoxyphényldimédonyliodone, p-nitrophényldimédonyliodone, p-chlorophényldimédonyliodone, p-bromophényldimédonyliodone, phényldibenzoylméthyliodone ou p-biphénylyldimédonyliodone.

10. Composition de la revendication 8, dans laquelle le composé est l'o-nitrophénylisopropylidènemalonyliodone de formule